# EUROPÄISCHE PATENTSCHRIFT

(11) **EP 2 723 413 B1**
(45) Veröffentlichungstag und Bekanntmachung des Hinweises auf die Patenterteilung: **25.10.2017**
(21) Anmeldenummer: 12728368.7
(22) Anmeldetag: 14.06.2012
(51) Int. Cl.: A61M 1/00

(54) **SYSTEM ZUM ABSAUGEN VON FLUIDEN AUS EINEM KÖRPER MITTELS UNTERDRUCK**
SYSTEM FOR ASPIRATING FLUIDS FROM A BODY BY MEANS OF A VACUUM
SYSTÈME DESTINÉ À ASPIRER DES FLUIDES HORS D'UN CORPS PAR UN EFFET DE DÉPRESSION

(30) Priorität: 23.06.2011 CH 10642011
(43) Veröffentlichungstag der Anmeldung: 30.04.2014
(73) Patentinhaber: Medela Holding AG, 6340 Baar (CH)
(72) Erfinder: EHLERT, Hilmar, CH-6052 Hergiswil (CH)
(74) Vertreter: Clerc, Natalia
(86) Internationale Anmeldenummer: PCT/CH2012/000132
(87) Internationale Veröffentlichungsnummer: WO 2012/174672

(56) Entgegenhaltungen:
- WO-A1-2004/037334
- WO-A2-2009/089390
- US-A1- 2010 204 663

## Beschreibung

### TECHNISCHES GEBIET

Die vorliegende Erfindung betrifft ein System zum Absaugen von Fluiden aus einem menschlichen oder tierischen Körper mittels Unterdruck gemäss Oberbegriff des Patentanspruchs 1.

### STAND DER TECHNIK

Zum Absaugen von Körperflüssigkeiten bzw. -fluiden im medizinischen Bereich, beispielsweise bei der Thoraxdrainage oder bei der Wunddrainage, aber auch während bzw. nach chirurgischen Eingriffen oder beim Absaugen von Körperfetten, werden Drainagepumpsysteme eingesetzt. Diese Systeme weisen üblicherweise eine Saug- oder Vakuumpumpe, einen oder mehrere Fluidsammelbehälter und eine Drainageschlauchverbindung zwischen Patient und Fluidsammelbehälter auf.

Durch Erzeugen eines Unterdrucks im Fluidsammelbehälter wird Fluid oder Sekret von einer Kavität des Patienten über den Drainageschlauch in den Sammelbehälter gesaugt und dort gesammelt. Am pumpenseitigen Ausgang des Sammelbehälters angeordnete Filter schützen die Saugpumpe vor einer allfälligen Verunreinigung durch das abgesaugte Fluid.

Der Fluidsammelbehälter kann mit der Pumpe über einen externen Vakuumschlauch und über ein Wasserschloss verbunden sein, wie dies beispielsweise in WO 03/103747 offenbart ist. Er kann jedoch auch am Gehäuse der Drainagepumpe lösbar befestigt sein, wie dies beispielsweise in US 2009/0163882 und US 2009/0157019 vorgeschlagen ist. In diesem Fall verläuft die Vakuumleitung im Innern des Pumpengehäuses.

Es ist ferner bekannt, zusätzlich zur Drainageleitung eine Serviceleitung von der Pumpe zum Patienten zu führen. In US 5 738 656 wird beispielsweise ein doppellumiger Schlauch verwendet, wobei ein erstes Lumen die Drainageleitung bildet und das zweite Lumen eine Luftleitung ist, welche am patientenseitigen Ende in die Drainageleitung mündet. Dadurch kann in die abzusaugende Kavität des Patienten Luft oder Gas zugeführt werden und so die Kavität gespült werden. Zudem kann dieses Lumen als Messleitung benützt werden, um Strömungs- oder Druckdifferenzen festzustellen.

In WO 2005/061025 wird ebenfalls eine mit dem patientenseitigen Ende des Drainageschlauchs verbundene Serviceleitung zum Spülen der Drainageleitung verwendet, um Verschlüsse der Leitung durch angesaugte Fluidklumpen oder Gewebe zu vermeiden bzw. zu beheben.

Auch US 2011/0071415 zeigt einen Drainageschlauch und eine separate Spülleitung.

WO 2010/126444 und WO 2011/037524 betreffen ein Wunddrainagesystem mit einer Spülleitung und einer zusätzlichen Pumpe. Diese zweite Pumpe wird dazu verwendet, Luft vom Fluidsammelbehälter durch die Spülleitung zur Wunde und von dort durch die Drainageleitung zurück in den Fluidsammelbehälter zu saugen.

WO 2009/089390 offenbart ein Drainagesystem mit einem Luftdrucktank, in welchem Abluft der Pumpe gespeichert und über eine Leitung in die Wunde eingebracht wird um einen sich rasch ändernden Saugzyklus zu erzielen.

WO 2004/037334 beschreibt eine Vorrichtung zur Reinigung von Wunden, wobei eine Flüssigkeit von einem Reservoir und Wundflüssigkeit mittels einer Pumpe einer Reinigungseinheit zugeführt und die gereinigte Körperflüssigkeit wieder in die Wunde zurücktransportiert wird.

### DARSTELLUNG DER ERFINDUNG

Es ist eine Aufgabe der Erfindung, ein verbessertes System zum Absaugen von Fluiden zu schaffen.

Diese Aufgabe löst ein System mit den Merkmalen des Patentanspruchs 1.

Das erfindungsgemässe System zum Absaugen von Fluiden aus einem Körper mittels Unterdruck weist auf: eine Drainageleitung zum Abführen des Fluids aus dem Körper, einen mit der Drainageleitung verbundenen Fluidsammelbehälter zum Sammeln des abgesaugten Fluids, eine Pumpe zur Erzeugung des zum Absaugen des Fluids eingesetzten Unterdrucks in der Drainageleitung sowie eine Hilfsleitung, deren körpernahes Ende mit einem körpernahen Ende der Drainageleitung in fluidkommunizierender Verbindung steht. Erfindungsgemäss ist im System ein geschlossener oder annähernd geschlossener Kreislauf vorhanden oder erzeugbar, welcher diese Pumpe, die Hilfsleitung, die Drainageleitung und den Fluidsammelbehälter miteinander verbindet und durch welchen Luft oder eine Flüssigkeit pumpbar ist, wobei die Hilfsleitung zur Spülung des Drainageschlauchs verwendbar ist.

Vorzugsweise ist zur Bildung des geschlossenen oder annähernd geschlossenen Kreislaufs ein Zweiwegventil im System vorhanden, wobei das Zweiwegventil eine erste Stellung aufweist, in welcher die Verbindung zwischen Pumpe und Hilfsleitung unterbrochen ist, so dass durch die Pumpe abgesaugte Luft in die Umgebung abgegeben wird und der Kreislauf offen ist. In dieser Stellung wird im Fluidsammelbehälter und in der Drainageleitung ein Unterdruck aufgebaut und so das Körperfluid aus der Körperöffnung abgesaugt. In einer zweiten Stellung des Zweiwegventils ist die Verbindung zwischen Pumpe und Hilfsleitung geschlossen und der erfindungsgemässe annähernd geschlossene Kreislauf ist gebildet.

Der Kreislauf ist üblicherweise lediglich annähernd geschlossen, weil die Drainageleitung in einem Anschlussstück endet, welche Saugöffnungen zur Wunde hin aufweist, durch welche Wundflüssigkeit abgesaugt werden kann. Die Drainageleitung und die Hilfsleitung enden in einem gemeinsamen Katheter oder einem anderen gemeinsamen Anschlussstück. Derartige Katheter und Anschlussstücke sind aus dem Stand der Technik bekannt.

Die Drainagepumpeinheit zur Verwendung in einem erfindungsgemässen System weist eine Saugpumpe auf zur Erzeugung eines zum Absaugen eines Fluids eingesetzten Unterdrucks, welcher in einer mit der Drainagepumpeinheit verbindbaren Drainageleitung anlegbar ist. Die Drainagepumpeinheit ist mit einer Hilfsleitung verbindbar, deren körpernahes Ende mit einem körpernahen Ende der Drainageleitung in fluidkommunizierender Verbindung steht. Erfindungsgemäss weist die Drainagepumpeinheit ferner ein Zweiwegventil auf, wobei das Zweiwegventil in einer ersten Stellung zur Entlüftung der Saugpumpe in die Umgebung und in einer zweiten Stellung zur Verbindung der Saugpumpe mit der Hilfsleitung dient. In dieser zweiten Stellung des Zweiwegventils ist die Hilfsleitung zur Spülung des Drainageschlauchs verwendbar.

Diese Drainagepumpeinheit kann die Saugpumpe und deren Steuerung umfassen. Vorzugsweise umfasst sie auch einen oder mehrere Fluidsammelbehälter, welche am Gehäuse der Saugpumpe befestigbar sind. Vorzugsweise ist diese Drainagepumpeinheit als tragbares Gerät ausgebildet. In einem Verfahren zum Betreiben eines derartigen Drainagesystems wird eine Verbindung zwischen der Pumpe und der Hilfsleitung geschaffen, durch welche Luft aus der Pumpe durch die Hilfsleitung strömt. Dadurch wird ein annähernd geschlossener Kreislauf erzeugt, welcher diese Pumpe, die Hilfsleitung, die Drainageleitung und den Fluidsammelbehälter miteinander verbindet, wobei diese Luft durch die Drainageleitung in den Fluidsammelbehälter gepumpt wird, wodurch die Drainageleitung gereinigt wird. Alternativ kann auch eine Flüssigkeit von der Pumpe durch die Hilfsleitung gepumpt werden, um die Drainageleitung zu reinigen. Als derartige Flüssigkeit eignet sich beispielsweise eine Salzlösung.

Ist der Kreislauf bis auf die erwähnten Öffnungen im Katheter bzw. im Anschlussstück geschlossen, kann die Hilfsleitung zur Spülung des Systems, insbesondere des Drainageschlauchs, verwendet werden. Luft, welche die Vakuumpumpe bei geöffnetem Kreislauf in die Umgebung abgibt, wird nun in die Hilfsleitung gepumpt. Sind Hilfsleitung und Drainageleitung patientenseitig miteinander in fluidkommunizierender Verbindung, so spült die Luft die Drainageleitung und gelangt in den Fluidsammelbehälter. Flüssigkeiten in der Drainageleitung werden dabei zum Fluidsammelbehälter abtransportiert. Das Spülen der Drainageleitung mit Luft oder einer Flüssigkeit verhindert frühzeitig ein Verstopfen des Systems, weil Sekret regelmässig aus dem Drainageschlauch und allfälligen Kopplungsteilen entfernt wird und sich so keine eingetrockneten Fluid-Säulen bilden können.

Dank des Filters im Fluidsammelbehälter wird eine Verschmutzung der Saugpumpe nicht nur während des Drainagevorgangs sondern auch beim Spülen des Systems verhindert. Verfügt auch die Hilfsleitung über ein oder mehrere Filter, so ist auch diesseits eine Verschmutzung der Pumpe vermieden. Dieses zweite Filter verhindert zudem, dass Partikel oder Verunreinigungen von der Pumpe zur Wunde gelangen können.

Vorteilhaft an diesem erfindungsgemässen System ist, dass eine einzige Pumpe für die Drainage wie auch für die Spülung verwendet werden kann. Dies reduziert die Herstellungskosten. Die Drainagepumpeinheit lässt sich dank der einzigen Pumpe relativ klein und leicht ausbilden, was insbesondere bei einem tragbaren System vorteilhaft ist. Auf einem eingestellten Druckniveau wird in der zweiten Stellung Luft zirkuliert. Das Druckniveau bleibt während der Spülung in etwa gleich hoch. Dieses Druckniveau kann dem Druckniveau während des Absaugprozesses entsprechen. Die Geschwindigkeit der Pumpe steuert die Geschwindigkeit (Intensität) des Luftstroms ohne das Druckniveau zu beeinflussen.

Für die Spülung wird keine externe Luft verwendet. Dies ist insbesondere in Spitalumgebung wünschenswert, da das Risiko, externe Keime in die Wunde einzubringen, minimiert ist. Es ist jedoch auch möglich, anstelle von Luft eine Flüssigkeit durch die Hilfsleitung zur Drainageleitung zu pumpen, um so das System zu reinigen.

Es ist eine weitere Aufgabe der Erfindung, ein System zu schaffen, mittels welchem ein Druck im Bereich der Körperkavität gemessen werden kann.

Auch diese Aufgabe lösen ein System mit den Merkmalen des Patentanspruchs 1 Da ein Drucksensor vorhanden ist, lässt sich auf einfache Art und Weise der Druck im Bereich der Wunde oder einer anderen Körperkavität feststellen. Hierfür wird die Drainageleitung zuerst wie oben beschrieben gespült und anschliessend wird bei abgeschalteter Pumpe der Druck in der Drainageleitung und somit auch in der Kavität des Körpers gemessen. Der Drucksensor kann hierfür am behälterseitigen Ausgang der Pumpe, zwischen Fluidsammelbehälter und Pumpe, im Fluidsammelbehälter oder in der Drainageleitung angeordnet sein. Der Sensor ist zwischen Pumpe und Fluidsammelbehälter angeordnet. Es ist somit nicht notwendig, einen Sensor in die Nähe der Wunde oder im Bereich der Hilfsleitung anzuordnen. Es lässt sich derselbe Drucksensor verwenden, welcher auch den Betrieb der Pumpe während des Drainagevorgangs überwacht.

Des Weiteren lässt sich dank des erfindungsgemässen Systems eine Verstopfung einfach erkennen. Steigt beim Spülen der Druck am Drucksensor an, so ist der geschlossene Kreislauf nicht frei und eine Verstopfung muss behoben werden.

Vorzugsweise erfolgt die Spülung und ebenfalls vorzugsweise die Druckmessung automatisch und sich wiederholend. Die entsprechende Steuerung des Zweiwegventils kann über die Steuerung der Pumpe erfolgen. Auch ist es vorteilhaft, dass lediglich ein Ventil betätigt werden muss und nicht noch weitere Pumpen oder andere Bauteile involviert sind. Diese Vereinfachung reduziert Entwicklungs- und Herstellungskosten, erhöht die Funktionssicherheit im Gebrauch und reduziert allfällige Wartungsarbeiten.

Das erfindungsgemässe System entfernt somit erfolgreich Sekretverstopfungen aus dem Schlauchsystem, bietet eine aktive Prävention von Verstopfungen an, detektiert bestehende Verstopfungen und ermöglicht eine relativ genaue Druckmessung im Bereich der Körperkavität bzw. der Wunde.

Das erfindungsgemässe System ist insbesondere als Wunddrainagesystem, als Cardiodrainagesystem und als Thoraxdrainagesystem geeignet. Die eingangs erwähnten weiteren Anwendungsbereiche sind jedoch auch möglich.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: eine schematische Darstellung des erfindungsgemässen Systems in Verwendung bei einer Wunddrainage und
- Figur 2: eine schematische Darstellung des erfindungsgemässen System in Verwendung einer Thorax- und Cardiodrainage.

### BESCHREIBUNG BEVORZUGTER AUSFÜHRUNGSFORMEN

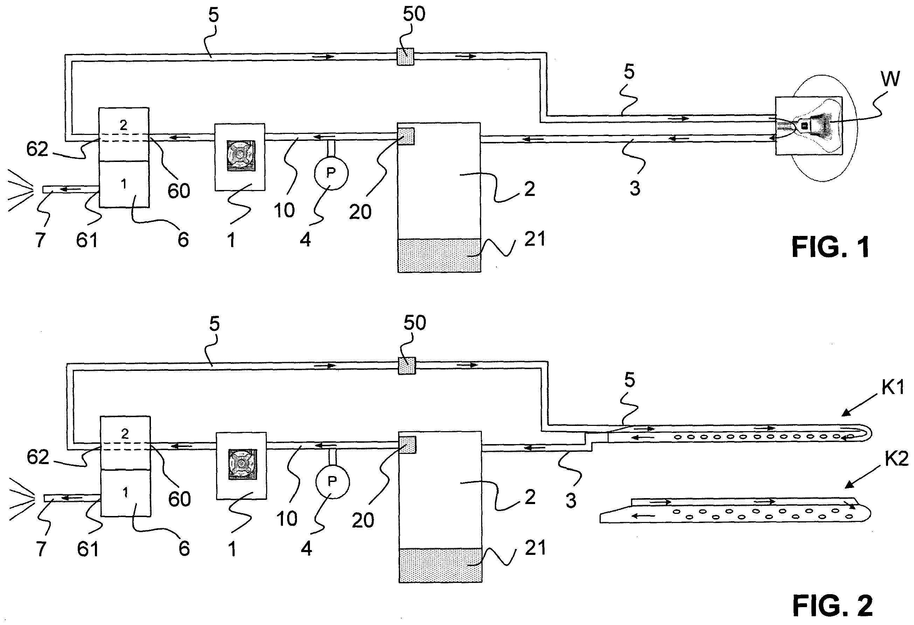

In Figur 1 ist das erfindungsgemässe System während einer Wunddrainage dargestellt.

Das System weist eine Saugpumpe 1 auf, vorzugsweise eine motorbetriebene elektrische Vakuumpumpe, insbesondere eine Membranpumpe bekannter Art. Über eine Vakuumleitung 10 ist ein Fluidsammelbehälter 2 mit einem ersten Ausgang der Saugpumpe 1 verbunden, so dass ein mittels der Pumpe erzeugter Unterdruck im Behälter 2 angelegt wird. Am pumpenseitigen Eingang des Fluidsammelbehälters 2 ist mindestens ein Filter 20 angeordnet, um eine Verunreinigung der Pumpe 1 zu vermeiden. Das Filter 20 ist bekannter Art, insbesondere ist es ein Überlauf- und/oder Bakterien- und/oder Geruchsfilter. Der Behälter 2 kann sich ausserhalb des Pumpengehäuses befinden oder er kann an diesem lösbar befestigt sein.

Eine Drainageleitung 3 mündet in den Fluidsammelbehälter 2, wobei diese Leitung 3 das Innere des Behälters 2 mit einer Kavität eines menschlichen oder tierischen Körpers verbindet. Die Kavität ist in diesem Beispiel eine Wunde, auf welcher eine Wundabdeckung W befestigt ist. Die Drainageleitung 3 führt in diese Wundabdeckung W hinein bzw. sie ist an einem daran angeordneten Kopplungsstück befestigt. Als Drainageleitung 3 eignen sich insbesondere die bekannten Medizinalschläuche, beispielsweise aus Silikon.

Die Drainageleitung 3 kann mittels eines geeigneten Anschlussteils im Behälter 2 eingesteckt sein. Sie kann jedoch auch, wie aus dem oben genannten Stand der Technik bekannt ist, über ein geeignetes Kopplungsteil im Gehäuse der Pumpe 1 eingesteckt und mit dem Behälter 2 lösbar verbunden sein.

Zwischen Fluidsammelbehälter 2 und Pumpe 1 ist ein Drucksensor 4 zur Messung des Drucks in der Vakuumleitung 10 und somit auch in der Drainageleitung 3 angeordnet. Der Sensor 4 kann auch an einer anderen Stelle angeordnet sein, solange seine Lage dazu geeignet ist, den Druck in der Drainageleitung 3 zu messen.

Die Pumpe 1 ist an einem zweiten Ausgang mit einem Eingang 60 eines Zweiwegventils 6 verbunden. In einer ersten Stellung des Zweiwegventils 6 ist eine Verbindung vom Eingang 60 zu einem ersten Ausgang 61 des Ventils 6 geschaffen. Dieser Ausgang 61 bildet die Entlüftungsöffnung bzw. den Auspuff des Systems oder er führt über eine Entlüftungsleitung 7 zu einem derartigen Auspuff.

In dieser ersten Stellung des Zweiwegventils 6 arbeitet die Pumpe als Vakuumpumpe, wobei ein Unterdruck im Fluidsammelbehälter 2 und in der Drainageleitung 3 angelegt und Fluide und Sekret aus der Wunde in den Behälter 2 gesogen werden. Das abgesaugte Sekret ist in Figur 1 mit der Bezugsziffer 21 versehen.

Das Zweiwegventil 6 weist einen zweiten Ausgang 62 auf. An diesen ist eine Hilfsleitung 5, auch Spülleitung genannt, angeschlossen. Diese Hilfsleitung 5 führt ebenfalls zur Wunde, wobei sie an ihrem wundseitigen Ende mit dem wundseitigen Ende der Drainageleitung 3 in fluidkommunizierender Verbindung steht. Beide dieser Enden gehen in ein gemeinsames wundseitiges Anschlussstück über. Derartige Anschlussstücke sind aus dem Stand der Technik bekannt. Die Hilfsleitung 5 und die Drainageleitung 3 können zwei vollständig getrennt voneinander verlaufende Schläuche, insbesondere aus Silikon sein. Es ist jedoch auch möglich, dass die zwei Schläuche wenigstens abschnittsweise aneinander befestigt sind. Vorzugsweise wird jedoch ein doppellumiger Schlauch verwendet, wobei ein erstes Lumen die Drainageleitung 3 und ein zweites Lumen die Hilfsleitung 5 bildet. Die Hilfsleitung 5 weist vorzugsweise einen kleineren inneren Durchmesser auf als die Drainageleitung 3. Die Hilfsleitung 5 kann auch noch anderen Zwecken dienen, beispielsweise zum Einbringen von Flüssigkeiten und Medikamenten in die Kavität. Hierzu kann ein hier nicht dargestellter separater und verschliessbarer Eingang in der Hilfsleitung 5 vorhanden sein.

Vorzugsweise ist in der Spülleitung 5 mindestens ein Filter 50 bekannter Art angeordnet, um eine Verschmutzung der Pumpe bzw. der Wunde zu verhindern. Das Filter 50 kann am zweiten Ausgang 62 des Zweiwegventils 6 angeordnet sein.

Als Zweiwegventil 6 eignen sich bekannte Ventile, welche vorzugsweise elektronisch betätigbar sind und welche einen Eingang und zwei wahlweise und abwechslungsweise mit dem Eingang verbindbare Ausgänge aufweisen.

Befindet sich nun das Zweiwegventil 6 in seiner zweiten Stellung, so ist die Verbindung zwischen dem Eingang 60 und dem ersten Ausgang 61 unterbrochen, dafür aber die Verbindung zwischen Eingang 60 und zweitem Ausgang 62 erstellt. Es wird dadurch ein geschlossener Kreislauf der Leitungen 3, 5, 10 gebildet. Lediglich die Öffnungen des Katheters bzw. des Anschlussstücks sind noch vorhanden. Der Kreislauf erstreckt sich von der Vakuumpumpe 1 über das Zweiwegventil 6 und über die Hilfsleitung 5 zum wundseitigen Ende der Hilfsleitung 5 und von dort über die Drainageleitung 3 zurück zum Fluidsammelbehälter 2, zur Vakuumleitung 10 und zur Pumpe 1. Je nach Art des wundseitigen Anschlussstücks kann sich der Übergang von der Hilfsleitung 5 zur Drainageleitung 3 in einem kleinen Abstand vom Ende der Drainageleitung 3 oder direkt an diesem Ende befinden.

Ist nun dieser Kreislauf geschlossen, so erfolgt die Entlüftung der Vakuumpumpe 1 nicht mehr in die Umgebung sondern in die Hilfsleitung 5. Diese Druckluft kann dazu verwendet werden, um das System zu spülen und zu reinigen. Der Kreislauf der Luft ist in Figur 1 mit Pfeilen dargestellt. Die durch die Hilfsleitung 5 gepumpte Luft oder Flüssigkeit gelangt durch die Drainageleitung 3 in den Fluidsammelbehälter 2, wobei allfällig stecken gebliebene Partikel und Tropfen mitgesogen werden. Ist die Leitung verstopft, so detektiert der Drucksensor 4 einen Druckanstieg und meldet dies einem Überwachungssystem der Steuerung der Pumpe 1.

Nach der Spülung im geschlossenen Kreislauf kann eine Druckmessung in der Kavität, insbesondere in der Wunde durchgeführt werden. Hierzu wird die Pumpe 1 gestoppt und der Druck wird mit dem pumpeninternen Drucksensor 4 statisch gemessen.

Die Spülung erfolgt vorzugsweise kontinuierlich oder periodisch bei verschiedenen Luft-bzw. Flüssigkeitsgeschwindigkeiten. Die Spülzeit kann, insbesondere im periodischen Betrieb, einige Sekunden bis wenige Minuten betragen. Die Spülung kann von einem Benützer manuell ausgelöst werden. Vorzugsweise ist die Steuerung des Systems jedoch so ausgebildet, dass während eines Drainagevorgangs in definierten Abständen wiederholt automatisch gespült wird.

In Figur 2 ist das erfindungsgemässe System in einer Anwendung in der Thorax- oder Cardiodrainage dargestellt. Das System ist dasselbe. Gleiche Teile sind mit gleichen Bezugszeichen versehen. Die Drainageleitung 3 und die Hilfsleitung 5 enden hier jedoch in einem Katheter. In der Figur sind zwei verschiedene Katheterarten K1 und K2 dargestellt. Andere Arten von Kopplungsstücken oder Kathetern lassen sich ebenfalls verwenden.

Das erfindungsgemässe System verhindert und entfernt Verstopfungen in der Drainageleitung und ermöglicht zudem eine relativ genaue Messung des Unterdrucks in der Körperkavität.

**BEZUGSZEICHENLISTE**

| | | | |
|---|---|---|---|
| 1 | Saugpumpe | 6 | Zweiwegventil |
| 10 | Vakuumleitung | 60 | Eingang |
| 2 | Fluidsammelbehälter | 61 | erster Ausgang |
| 20 | Filter | 62 | zweiter Ausgang |
| 21 | abgesaugtes Sekret | 7 | Entlüftungsleitung |
| 3 | Drainageleitung | W | Wundabdeckung |
| 4 | Drucksensor | K1 | erster Katheter |
| 5 | Spülleitung | K2 | zweiter Katheter |
| 50 | Filter | | |

## Patentansprüche

1. System zum Absaugen von Fluiden aus einem menschlichen oder tierischen Körper mittels Unterdruck, wobei das System aufweist: eine Drainageleitung (3) zum Abführen des Fluids aus dem Körper, einen mit der Drainageleitung (3) verbundenen Fluidsammelbehälter (2) zum Sammeln des abgesaugten Fluids, eine Pumpe (1) zur Erzeugung des zum Absaugen des Fluids eingesetzten Unterdrucks in der Drainageleitung (3) sowie eine Hilfsleitung (5), deren körpernahes Ende mit einem körpernahen Ende der Drainageleitung (3) in fluidkommunizierender Verbindung steht, **dadurch gekennzeichnet, dass** die Drainageleitung (3) und die Hilfsleitung (5) in einem gemeinsamen Katheter oder einem anderen gemeinsamen Anschlussstück enden, dass im System ein annähernd geschlossener Kreislauf vorhanden oder erzeugbar ist, welcher diese Pumpe (1), die Hilfsleitung (5), die Drainageleitung (3) und den Fluidsammelbehälter (2) miteinander verbindet und durch welchen Luft oder eine Flüssigkeit pumpbar ist, dass die Hilfsleitung (5) zur Spülung des Drainageschlauchs (3) verwendbar ist, und dass zwischen der Pumpe (1) und dem Fluidsammelbehälter (2) ein Drucksensor (4) angeordnet ist, um den Druck in der Drainageleitung (3) zu messen.

2. System nach Anspruch 1, wobei das System ein Zweiwegventil (6) zur Bildung des annähernd geschlossenen Kreislaufes aufweist.

3. System nach Anspruch 2, wobei das Zweiwegventil (6) eine erste Stellung (61) aufweist, in welcher die Verbindung zwischen Pumpe (1) und Hilfsleitung (5) unterbrochen ist, so dass durch die Pumpe (1) abgesaugte Luft in die Umgebung abgegeben wird und der Kreislauf offen ist.

4. System nach Anspruch 3, wobei das Zweiwegventil (6) eine zweite Stellung (62) aufweist, in welcher die Verbindung zwischen Pumpe (1) und Hilfsleitung (5) geschlossen ist und der Kreislauf geschlossen ist.

5. System nach einem der Ansprüche 1 bis 4, wobei die Drainageleitung (3) im Fluidsammelbehälter (2) endet und die Pumpe (1) den Unterdruck im Fluidsammelbehälter (2) erzeugt.

6. System nach einem der Ansprüche 1 bis 5, wobei ein Filter (20) am pumpenseitigen Ausgang des Fluidsammelbehälters (2) angeordnet ist.

7. System nach einem der Ansprüche 1 bis 6, wobei die Hilfsleitung (5) ein Filter (50) aufweist.

8. System nach einem der Ansprüche 1 bis 7, wobei die Pumpe (1) eine Membranvakuumpumpe ist.

## Claims

1. System for aspirating fluids from a human or animal body by means of negative pressure, which system has: a drainage line (3) for removing the fluid from the body, a fluid collection container (2) connected to the drainage line (3) for collecting the aspirated fluid, a pump (1) for generating the negative pressure in the drainage line (3) to aspirate the fluid, and an auxiliary line (5) whose end near the body is in fluidic communication with an end, near the body, of the drainage line (3), **characterized in that**, the drainage line (3) and the auxiliary line (5) end in a common catheter or in a common connector piece, that, in the system, an approximately closed circuit is present or can be generated, which interconnects this pump (1), the auxiliary line (5), the drainage line (3) and the fluid collection container (2) and through which air or a liquid can be pumped, that the auxiliary line (5) is usable for flushing the drainage hose (3), and that a pressure sensor (4) is arranged between the pump (1) and the fluid collection container (2), in order to measure the pressure in the drainage line (3).

2. System according to Claim 1, wherein the system has a two-way valve (6) for forming the approximately closed circuit.

3. System according to Claim 2, wherein the two-way valve (6) has a first position (61), in which the connection between pump (1) and auxiliary line (5) is interrupted, such that air aspirated through the pump (1) is released into the environment and the circuit is open.

4. System according to Claim 3, wherein the two-way valve (6) has a second position (62), in which the connection between pump (1) and auxiliary line (5) is closed and the circuit is closed.

5. System according to one of Claims 1 to 4, wherein the drainage line (3) ends in the fluid collection container (2), and the pump (1) generates the negative pressure in the fluid collection container (2).

6. System according to one of Claims 1 to 5, wherein a filter (20) is arranged at the pump-side outlet of the fluid collection container (2).

7. System according to one of Claims 1 to 6, wherein the auxiliary line (5) has a filter (50).

8. System according to one of Claims 1 to 7, wherein the pump (1) is a diaphragm vacuum pump.

## Revendications

1. Un système pour l'aspiration de fluides hors d'un corps humain ou animal par l'intermédiaire de sous-pression, où le système présente: une conduite de drainage (3) pour l'évacuation du fluide hors du corps, un récipient de collecte de fluide connecté à la conduite de drainage (3) pour la collecte du fluide aspiré, une pompe (1) pour la création de la sous-pression utilisée pour l'aspiration du fluide dans la conduite de drainage (3) ainsi qu'une conduite auxiliaire (5), dont la partie terminale proche du corps est en connexion fluidiquement communicante avec une partie terminale proche du corps de la conduite de drainage (3), **caractérisé en ce que** la conduite de drainage (3) et la conduite auxiliaire (5) se terminent dans un cathéter commun ou une autre pièce de raccordement commune, que dans le système un système circulatoire approximativement fermé est présent ou peut être créé, lequel connecte cette pompe (1), la conduite auxiliaire (5), la conduite de drainage (3) et le récipient de collecte de fluide (2) entre eux et à travers duquel de l'air ou un liquide peut être pompé, que la conduite auxiliaire (5) peut être utilisée pour un rinçage de la conduite de drainage (3) et qu'un capteur de pression (4) est disposé entre la pompe (1) et le récipient de collecte de fluide (2) pour mesurer la pression dans la conduite de drainage (3).

2. Système selon la revendication 1, où le système présente une soupape à deux voies (6) pour la formation du système circulatoire approximativement fermé.

3. Le système selon la revendication 2, où la soupape à deux voies (6) présente une première position (61), dans laquelle la connexion entre la pompe et la conduite auxiliaire (5) est interrompue, de sorte que l'air aspiré par la pompe (1) est émis dans l'environnement et le système circulatoire est ouvert.

4. Le système selon la revendication 3, où la soupape à deux voies (6) présente une deuxième position (62), dans laquelle la connexion entre la pompe (1) et la conduite auxiliaire (5) est fermée et le système circulatoire est fermé.

5. Le système selon une des revendications 1 à 4, où la conduite de drainage (3) se termine dans le récipient de collecte de fluide (2) et la pompe (1) crée la sous-pression dans le récipient de collecte de fluide (2).

6. Le système selon une des revendications 1 à 5, où un filtre (20) est disposé au niveau de la sortie proche de la pompe du récipient de collecte de fluide (2).

7. Le système selon une des revendications 1 à 6, où la conduite auxiliaire (5) présente un filtre (50).

8. Le système selon une des revendications 1 à 7, où la pompe (1) est une pompe à vide à membrane.
